# EUROPEAN PATENT SPECIFICATION

(11) **EP 3 033 118 B1**
(45) Date of publication and mention of the grant of the patent: **18.10.2017**
(21) Application number: 14750184.5
(22) Date of filing: 06.08.2014
(51) Int. Cl.: A61M 1/00, A61M 1/06, F16K 15/14, F16K 24/06, G05D 16/06

(54) **SAFETY VALVE**
SICHERHEITSVENTIL
VALVE DE SÉCURITÉ

(30) Priority: 12.08.2013 WO PCT/CN2013/000950; 17.09.2013 EP 13184660
(43) Date of publication of application: 22.06.2016
(73) Proprietor: Koninklijke Philips N.V., 5656 AE Eindhoven (NL)
(72) Inventor: PAN, Yi Bing, NL-5656 AE Eindhoven (NL)
(74) Representative: Freeke, Arnold
(86) International application number: PCT/EP2014/066870
(87) International publication number: WO 2015/022244

(56) References cited:
- EP-A1- 1 479 521
- DE-U1-202006 012 645
- US-A- 6 024 120
- US-A1- 2003 188 790
- US-A1- 2008 255 503

## Description

### FIELD OF THE INVENTION

The present invention relates to a valve for controlling the flow of a medium from a first system to a second system. The present invention further relates to a pump device and to a breast pump device comprising a valve.

### BACKGROUND OF THE INVENTION

Safety valve mechanisms usually refer to mechanical or electrical components allowing to automatically release a medium from any kind of pressure vessel or other system when the pressure exceeds a preset limit. By releasing the pressure, the pressure vessel or system can be protected from being damaged or even from exploding. Further, any equipment or person being connected to this pressure vessel or system can also be protected from being harmed because of a too high or too low pressure.

Breast pump devices are usually used by lactating women for extracting milk from their breasts. Such breast pump devices may be manual devices powered by hand or foot movements or mains- or battery-powered electrical devices. Commonly, the milk is extracted by applying an underpressure to the breast. This underpressure is generated by a pump or a vacuum pump generating a (partial) vacuum in a milk reservoir being directly or indirectly connected to the breast. The collected milk flows into the milk reservoir wherein it is then stored. Depending on the type of the breast pump device, the device might be configured to provide a fluctuating vacuum profile for mimicking the sucking cycle of an infant. This fluctuating vacuum profile can be generated by controlling the pump appropriately or by making use of an appropriate valve construction.

One important aspect when designing a breast pump device is the safety of the user. Depending on the type of the used pump, it is possible that the pump generates a too high vacuum level causing harm or injury to the user. Possible causes therefor may be mechanical or electrical failure of single components, e.g. the pump or a solenoid valve. Consequently, breast pump devices are usually equipped with a safety valve. This safety valve is configured to open and release the vacuum if a vacuum limit is exceeded and to close again as soon as the vacuum drops below a limit again.

In US 2008/0177224 A1 there is disclosed a programmable electric breast pump system including a vacuum pump pneumatically coupled to breast cups and a vacuum relief valve bypassing the vacuum pump. This vacuum relief valve is cycled by means of a controller between minimum and maximum vacuum set points to create a periodic vacuum pulse in the breast cups, i.e. mimicking the sucking cycle of an infant. The relief valve also prevents the user from being harmed as the preset maximum vacuum level cannot be exceeded.

In US 6,024,120 A1 a pressure relief valve with moving diaphragm is disclosed. The valve comprises a diaphragm with an inner periphery and an outer periphery and an actuator having a piston extending axially therefrom which is inserted through the inner periphery of the diaphragm. To relieve an excess negative pressure condition the diaphragm is operable in one of two positions. In the first position, biasing members force the inner periphery of the diaphragm in sealing engagement around the circumference of the piston so that a hermetic seal is maintained. When negative pressure increases the inner periphery of the diaphragm moves in sealing engagement along the piston so that a constant sealing force is applied around the circumference of the piston until it reaches the plurality of legs at the free end of the piston. Thereby, ambient air is permitted to vent from one side of the valve to the other.

In EP 1 479 521 A1 an ink tank is disclosed. The ink tank is capable of providing a stable ink supply to a print head by using a simple valve construction. In one preferred embodiment, an ink path is closed by a lip portion of a valve rubber engaging a flange, and an angle formed by an inner surface of an opening in the lip portion and an engagement surface of the flange is an acute angle.

In DE 20 2006 012 645 U1 a check valve is disclosed. This valve is rotationally symmetric and has a valve seat containing a center section, an inlet channel and an outlet channel. A flexible seal covers the outlet channel forming a valve closing member. The center section is conically shaped and bent towards the outlet channel in an axial direction. The seal has an appropriate form. The seal is limited against and links to an upper section of the center section.

In US 2003/0188790 A1 an underpressure valve is disclosed. The valve has a multi-part housing and a disk-like closing body which has at least one opening, and with an opening-free section disposed in alignment with the flow channel of the housing.

However, conventional safety valves often have the problem that they open proportionally to the applied vacuum level, i.e. they open as soon as the maximum vacuum limit is exceeded but close again directly after the vacuum level has dropped below this preset maximum level again. Such a design can only guarantee that a maximum vacuum limit is not exceeded but can, however, not assure that the vacuum can be relieved if the system is out of control.

### SUMMARY OF THE INVENTION

It is thus an object of the present invention to overcome the above mentioned problems and to provide a safety valve allowing to return to a controlled setting of a pump, in particular a breast pump device, if the system is out of control. It is further an object of the present invention to provide an improved safety valve allowing a reliable control of the vacuum level. It is yet another object of the present invention to provide an efficiently producible valve. The invention is defined by the features of the independent claim. In a first aspect of the present invention, there is presented a valve for controlling the flow of a medium from a first system to a second system comprising a deformable dome-shaped structure forming a reservoir and including a flow path opening located in a zenith of a hemisphere part of the deformable dome-shaped structure, a valve cover including a first connector opening to connect the valve to the first system and a contact element for interacting with the flow path opening to open and close it and a valve base being airtightly connected to the valve cover and to the dome-shaped structure and including a second connector opening to connect the valve to the second system and being coupled to the reservoir, characterized in that the deformable dome-shaped structure is configured such that in an open state of the valve the deformable dome-shaped structure is deformed into a dented shape so that the flow path opening is not connected to the contact element to allow a medium to flow from the first system to the second system through the first and second connector openings and the flow path opening and in a closed state of the valve the deformable dome-shaped structure is in its original dome shape so that the flow path opening is connected to the contact element and thus closed.

In a further aspect of the present invention, there is presented a pump device for transporting a medium from a first system connected to its inlet to a second system connected to its outlet, wherein at least one valve as described above is interposed between at least one of the first system and the inlet of the pump and the second system and the outlet of the pump.

In yet another aspect of the present invention, there is presented a breast pump device for extracting milk from the breast of a lactating woman comprising a milk reservoir for collecting the extracted milk, a funnel connected to the milk reservoir for receiving a breast of a women, a vacuum pump for generating an underpressure in the milk reservoir and a valve as described above connected to the milk reservoir for preventing the pressure applied to the breast of the woman from exceeding a pressure release threshold and for providing a pressure fluctuation between the pressure release threshold and the pressure hold threshold mimicking the sucking cycle of an infant.

Depending on the application area of the valve according to the present invention, the flow of different medium can be controlled. For instance, a medium may refer to a gas such as air, oxygen, nitrogen or others, e.g. in medical applications. Further, a medium can also refer to a liquid, such as water, blood, fuel, beer or others, e.g. when transporting such liquids in pressurized containers through different temperature zones. Generally, a medium as used therein may refer to any other kind of fluid, i.e. gas or liquid.

The valve is thereby usually arranged in between two systems, the first system usually referring to the ambience, the second system usually referring to the pressurized tank for storing a vacuum level. For instance, one example could include a vacuum tank representing a second system being evacuated by means of a vacuum pump. This tank or any device or user being in contact with this tank is prevented from being harmed by means of a valve according to the present invention. The ambient air around the pressurized tank represents the first system. The valve allows connecting the two systems and thereby balance or gradually balance the pressure levels in the two systems.

The valve according to the present invention comprises a deformable dome-shaped structure, a valve cover and a valve base. These three components interact for providing the desired functionality. The deformable dome-shaped structure is thereby deformable, i.e. flexible in the sense that it can be deformed if force is applied but may also be reversed to its original form if the force is released or if a second (oppositely directed) force is applied. One advantage of the deformable dome-shaped structure is that no mechanical damage occurs if a force, i.e. a pressure or pressure difference, is applied to it. The valve cover includes a first connector opening forming the inlet side of the valve. This first connector opening allows the flow of a medium from the first system into the valve. There is further provided a contact element interacting with the flow path opening in the deformable dome-shaped structure in order to open and close it. This functionality can e.g. be realized by arranging the contact element such that it is pressed against the flow path opening or that the flow path opening is pressed against the contact element so that the flow path opening is sealed. If, however, the flow path opening is not connected to the contact element, a medium can flow through it.

The valve base is designed to interact with the valve cover. In particular, valve base and valve cover can be assembled such that the deformable dome-shaped structure is included in a housing formed by the valve cover and the valve base. The valve base further includes second connector openings which represent the connection of the valve to the second system. Thus, a medium can enter the valve from the second system through the second connector opening and vice versa. The valve base is mechanically designed such that the second connector opening directly connects the second system to the reservoir formed by the deformable dome-shaped structure. The valve base further comprises means for mechanically supporting and airtightly connecting the deformable dome-shaped structure. Thus, if a medium enters the valve through the second connector opening, it directly enters the reservoir formed by the deformable dome-shaped structure is only able to exit the deformable dome-shaped structure through the flow path opening. Depending on whether the valve is in open state, the flow path opening is not connected to the contact element and thus a medium can flow through it. Opposed to that, if the flow path opening is connected to the contact surface, the valve is in closed state and no medium can flow.

A particular advantage of the present invention is that depending on the pressure differences between the first and the second system the functionality of a safety valve can be realized. A medium can either enter the valve through the first connector opening, pass through the flow path opening and leave the valve through the second connector opening, or enter the valve through the second connector opening path through the flow path opening and leave the valve through the first connector opening. The flow path opening therein has the key role to allow the medium to flow or to block the flow path depending on the state of the valve.

According to one embodiment of the present invention, the deformable dome-shaped structure is deformed and the flow path opening is not connected to the contact element if the pressure difference between the first system and the second system is above a pressure release threshold and the deformable dome-shaped structure is in its original dome-shape and the flow path opening is in contact with the contact element if the pressure difference between the first system and the second system is below a pressure hold threshold.

Thus, according to this embodiment, the deformable dome-shaped structure is caused to be deformed, when a pressure difference between the first and the second system occurs. This deformable dome-shaped structure can only hold its original dome-shaped form if the force, i.e. the pressure, applied to it is below a certain pressure threshold, i.e. a pressure hold threshold. If the pressure difference is high, a deformation of the deformable dome-shaped structure is caused. The flow path opening is arranged such that such a deformation of the deformable dome-shaped structure causes it to loose its contact with the contact element. This brings the valve into open state and a medium can be exchanged between the two systems.

One particular advantage of this embodiment is that no external control, e.g. an electrical control, of the valve is needed. The valve can provide its functionality solely based on the pressure difference between the first and the second system. A further advantage of the present invention is that the valve's composition of merely three components, i.e. deformable dome-shaped structure, valve cover and valve base allows a cheap and efficient manufacturing of the valve as well as integration in a small volume.

The pressure release and hold thresholds thereby preferably depend on the mechanical design or form and on the material properties of the deformable dome-shaped structure.

According to another preferred embodiment of the present invention, the deformable dome-shaped structure is deformed and the flow path opening is disconnected from the contact element when the pressure difference between the first system and the second system exceeds a pressure release threshold and the deformable dome-shaped structure recovers to its original dome shape and the flow path opening is in contact with the contact element when the pressure difference between the first system and the second falls below a pressure hold threshold.

According to this embodiment, the valve functions dynamically. It is brought into open state if the pressure difference exceeds this first threshold. When the pressure difference falls under a threshold again, the deformable dome-shaped structure reverts to its original form such that the flow path opening is in contact with the contact element and the flow path is sealed. Depending on the configuration of the system, this may allow the pressure difference to increase again until the pressure release threshold is exceeded again.

According to a preferred embodiment of the present invention the pressure release threshold is higher than the pressure hold threshold and the valve is configured to provide a switching hysteresis. The pressure rises until the pressure release threshold is exceeded and the deformable dome-shaped structure is deformed such that the flow path opening is traversable and a medium can flow through the valve, i.e. through the first and second connector openings and the flow path opening. Then, as soon as medium flows, the pressure difference usually decreases again. According to this embodiment of the present invention, however, the deformable dome-shaped structure does not recover to its original dome shape until the pressure hold threshold is passed. If this pressure hold threshold is below the pressure release threshold, the valve thus provides a switching hysteresis in that, once open, the valve stays open for a certain time although the pressure is already below the threshold, initially causing the valve to open.

One advantage of this embodiment is that a delay can be achieved. Such a delay can, on the one hand, be used to configure a valve according to the present invention to provide a fluctuating pressure level, which could, e.g., be used in a breast pump device in order to mimic the sucking cycle of an infant. On the other hand, this behavior can also be exploited by providing a sort of safety functionality in that a good part of the vacuum is released before the vacuum level rises again. Thus, if e.g. a vacuum pump is out of control and provides a very high vacuum level, a user of a breast pump device comprising such a valve can be prevented from harm because the provided hysteresis allows him to take the breast pump device away during a low vacuum level period.

According to a preferred embodiment of the present invention, the pressure release threshold is between 250 mmHg and 450 mmHg, preferably between 330 mmHg and 360 mmHg, and/or the pressure hold threshold is between 50 mmHg and 200 mmHg, preferably between 90 mmHg and 120 mmHg. These values for the pressure release and pressure hold thresholds allow a gentle application of a breast pump device comprising a valve according to the present invention to a user. Other values for the thresholds are possible.

According to yet another embodiment of the present invention, the deformable dome-shaped structure comprises a polymer or elastomer material, in particular silicone or (liquid or solid) silicone rubber. By making use of such a polymer or elastomer material, the desired deformation functionality can be achieved. An elastomer material makes it possible to manufacture elastic parts that can be efficiently manufactured in virtually arbitrary designs. The deformable dome-shaped structure comprised in the valve according to the present invention may, e.g., be manufactured in a molding or injection molding process.

According to another embodiment of the present invention, the deformable dome-shaped structure includes a cylinder part and a hemisphere part, the hemisphere part comprising the flow path opening and having a diameter bigger or equal to the diameter of the cylinder part. Structuring the deformable dome-shaped structure such that it includes a cylinder part and a hemisphere part allows easily connecting the deformable dome-shaped structure to the valve base. Preferably, the flow path opening is located in the zenith of the hemisphere. Further preferably, the valve base comprises a cylinder-shaped dome connection appendix for receiving the cylinder part of the deformable dome-shaped structure and airtightly sealing the connection. If, e.g. the radius of the cylinder part of the deformable dome-shaped structure is a little bit smaller than the radius of the cylinder-shaped dome connection appendix, the airtight connection of the deformable dome-shaped structure with the cylinder-shaped dome connection appendix can be achieved by simply expanding the deformable dome-shaped structure and putting it over the cylinder-shaped dome connection appendix. Due to its material properties, an elastomer material, e.g. silicone or silicone rubber, can be connected without needing additional connection means such as glue. It is, however, also possible to use glue or other mechanical connection means such as clips or straps or others for airtightly connecting the deformable dome-shaped structure with the correspondent connection means or connection appendix of the valve base.

According to a preferred embodiment of the present invention, the flow path opening is arranged in the zenith of the deformable dome-shaped structure. Centering the flow path opening allows avoiding lateral forces on the deformable dome-shaped structure which could exert higher stress on the connection of the deformable dome-shaped structure with the valve base. Further, arranging the flow path opening in the zenith of the deformable dome-shaped structure also allows arranging the contact element opposite the flow path opening such that the tube parts are pressed together and the flow path opening is airtightly sealed.

According to yet another embodiment of the present invention, the contact element includes a flat surface area at least as big as the flow path opening in the deformable dome-shaped structure and arranged to seal the flow path opening in the deformable dome-shaped structure when pressed against the flat surface area. This flat surface area could be manufactured of the same material as the valve cover but could also comprise an alternative material such as an elastomer, e.g. silicone or silicone rubber. By providing a flat surface area at least as big as the flow path opening, this functionality can be provided.

According to yet another embodiment of the present invention, the first connector opening connecting the valve to the first system is represented by four venting holes arranged in a square around the contact element. Arranging the venting holes around the contact element and hereby the flow path opening allows a medium to flow smoothly from the first system into the valve or vice versa. Alternatively, it is possible to arrange more or less venting holes at other places in the valve cover if required by the application. If, e.g., the application requires the connection to the first system to be at a rectangle to the connection of the second system, this can be easily realized by providing venting holes at another position.

According to another embodiment, the second connector opening connecting the valve to the second system is represented by a cylinder-shaped system connection appendix connecting the reservoir to the second system. For the second connector opening the same reasoning holds as for the first connector opening. It is particularly advantageous to make use of a cylinder-shaped dome connection appendix. Such a cylinder appendix could be easily connected to a second system, e.g. by forming a screw head allowing to screw the valve into an appropriate screw hole for the connection with the second system. Preferably, this cylinder directly connects the reservoir formed by the deformable dome-shaped structure to the second system.

According to another embodiment of the present invention, the valve cover and the valve base are integrally formed. Integrally forming the two parts allows further decreasing manufacturing costs and increasing reliability of the part because no airtight connection of valve cover and valve base is required.

According to another aspect of the present invention, the pump device including a valve interposed between at least one of the first and/or the second system and the inlet and/or outlet of the pump can provide the advantage that automatically a back flow of medium through the pump is prevented. Thereby, the pump can be prevented from being harmed if malfunctioning occurs.

A breast pump device comprising a valve according to the present invention and as described above allows implementing the functionality of a safety valve. Even if the pump is not functioning appropriately and provides a too high level of vacuum, the valve allows preventing the user from being harmed. If such a situation occurs, the deformable dome-shaped structure is deformed and the vacuum is released to the ambience.

### BRIEF DESCRIPTION OF THE DRAWINGS

These and other aspects of the invention will be apparent from and elucidated with reference to the embodiments described hereinafter. In the following drawings
Fig. 1 shows a schematic illustration of a valve according to one embodiment of the present invention in exploded view;
Fig. 2 shows a sectional view of the valve in open state;
Fig. 3 shows a sectional view of the valve in closed state;
Fig. 4 shows a diagram illustrates the switching hysteresis provided by an embodiment of the present invention;
Fig. 5 shows a diagram that opposes a vacuum profile generated with an embodiment of the present invention to a vacuum profile generated with a conventional vacuum pump; and
Fig. 6 shows an embodiment of a breast pump device according to the present invention.

### DETAILED DESCRIPTION OF THE INVENTION

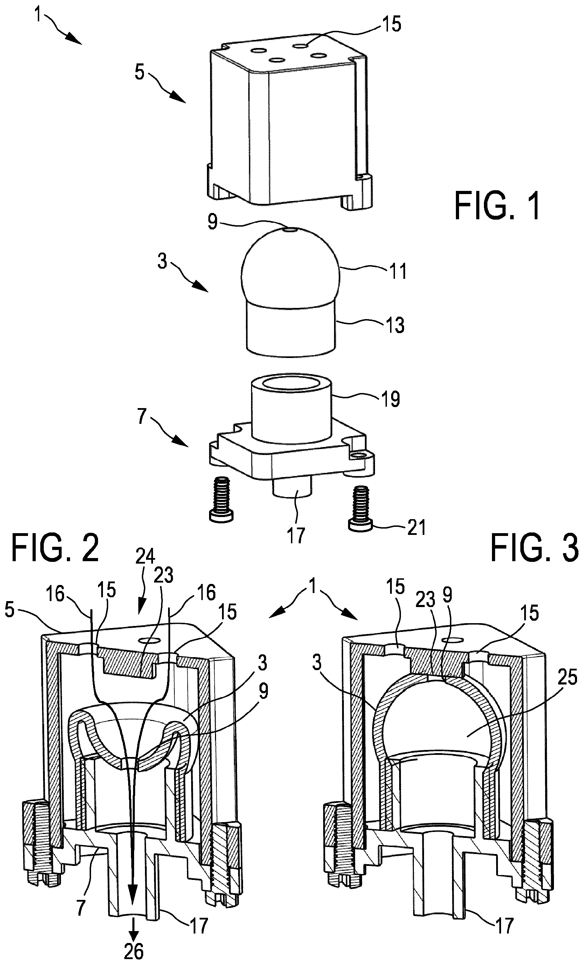

In Fig. 1 there is illustrated a valve 1 according to the present invention. The valve 1 comprises a deformable dome-shaped structure 3, a valve cover 5 and a valve base 7. In the illustrated example, the deformable dome-shaped structure 3 includes a flow path opening 9 arranged in the zenith of a hemisphere part 11 of the deformable dome-shaped structure 3. The structure 3 further comprises a cylinder part 13 having a diameter smaller than the diameter of the hemisphere part 11. The valve cover 5 includes a first connector opening 15 being represented by four venting holes arranged in a square on the upper side of the valve cover. The illustrated exploded view does not show the contact element. The valve base 7 includes a second connector opening 17 being represented by a cylinder-shaped system connection appendix. The second connector opening 17, respectively the cylinder-shaped system connection appendix, is formed to provide a support structure 19 for supporting the cylinder part of the deformable dome-shaped structure on its upper side.

Fig. 1 further shows two screws 21 for connecting valve base 7 and valve cover 5 and for forming a housing therefrom. According to one preferred embodiment of the present invention, valve cover 5 and valve base 7 are plastic parts manufactured in an injection die molding process. However, it would also be possible to use alternative materials and other means for materials for manufacturing the valve cover 5 and the valve base 7..

Fig. 2 illustrates the open state of an embodiment of a valve 1 according to the present invention. The deformable dome-shaped structure 3 is deformed such that the flow path opening 9 is not connected to the contact element 23. The contact element 23 is integrated with the valve cover 5 and manufactured from the same material. In the illustrated open state of the valve, a medium can flow through the conversed connector opening 15 pass through the flow path opening 9 and exit the valve, i.e. enter the second system through the second connector opening 17 comprised in the valve base 7, as indicated by the arrows 16. The medium may then flow from a first system 24 to a second system 26.

Fig. 3 illustrates an embodiment of the valve 1 according to the present invention in closed state. The flow path opening 9 is connected to the contact element 23 such that no medium can flow through the flow path opening and the flow path opening 9 is airtightly sealed. The deformable dome-shaped structure 3 is in its original dome-shaped form forming a reservoir 25. This reservoir is directly connected to the second system via the second connector opening 17.

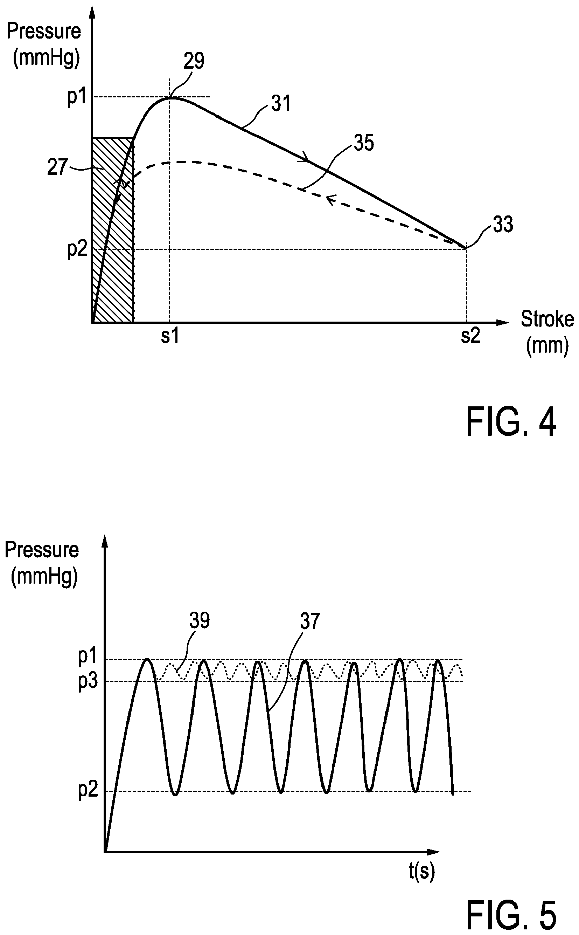

Fig. 4 illustrates the switching hysteresis that may be provided by an embodiment of a valve according to the present invention. On the x-axis, the stroke, i.e. the extent of the deformation of the deformable dome-shaped structure is indicated. On the y-axis, the pressure, i.e. the vacuum level causing the deformation is indicated. It can be seen that the dome deformation follows a kind of elastic deformation rule when the vacuum level in the second system increases. In normal operation, the vacuum level in the first system, respectively the pressure difference, is below the threshold and only little deformation occurs. The valve operates in normal working condition 27. If, however, the vacuum level leaves this normal working condition and increases above a pressure release threshold p1, the dome is deformed as soon as a yield point 29 is passed. The flow path opening is disconnected from the contact element (when the respective stroke s1 is reached) and the deformable dome shrinks along a shrink curve 31 although the vacuum level is already below the pressure release threshold p1. Because of the mechanical properties of the dome, even though the vacuum decreases below the pressure release threshold p1 due to leakage through the flow path opening, the dome keeps shrinking until a rebound point 33 is reached. This rebound point 33 corresponds to the pressure threshold p2 and the stroke s2. As soon as the rebound point 33 is passed, the dome deforms again along a rebound curve 35 and recovers to its original dome shape. Compared with a conventional safety valve, which opens proportional to the current vacuum level, the new valve according to the present invention opens for a longer time, i.e. provides a hysteresis, so that the vacuum can drop significantly.

In Fig. 5, the behavior of a valve according to the present invention is compared to the behavior of a conventional valve. The x-axis indicates the time, the y-axis indicates the pressure, i.e. the vacuum level. The vacuum profile 37 generated by an embodiment of a valve according to the present invention in interaction with a vacuum pump fluctuates between the pressure release threshold p1 and the pressure hold threshold p2. The deformable dome-shaped structure is deformed as soon as the pressure release threshold p1 is reached and the flow path opening is disconnected from the contact element. Once deformed, a medium can flow through the flow path opening and gradually balance the pressure level between the first and the second system. When the pressure hold threshold p2, i.e. a certain pressure force too small for holding the deformation, is reached, the deformable dome-shaped structure recovers, i.e. snaps, to its original dome shape and seals the flow path opening again. Then, the vacuum level can increase again until the pressure release threshold p1 is reached again.

In comparison to this behavior, the vacuum profile 39 of a conventional valve, which opens proportional to the currently applied vacuum level, shows a different behavior. If configured similarly to the illustrated example of the valve according to the present invention generating the vacuum profile 37, the conventional valve generating the vacuum profile 39 also releases the pressure when a pressure release threshold p1 is reached. However, as conventional valves only open proportional to the current pressure difference, the turning point is already reached at a pressure level p3 indicating a vacuum level higher than the pressure hold threshold p2. If, e.g., the system is in abnormal condition and the vacuum exceeds the (safety) limit a conventional valve can only keep the vacuum between the pressure release threshold p1 and the lower pressure level p3. At this vacuum level p3, however, the vacuum may already cause injuries, i.e., by sticking to a user's breast. The valve according to the present invention can relief the vacuum level down to the pressure release threshold allowing the user to pull off a breast pump device from the breast without causing any pain or injury.

The valve according to the present invention thus allows at least two different modus operandi. If the vacuum pump is operated under normal operating conditions, it will not generate a vacuum level close to the pressure release level defined by the mechanical properties of the deformable dome-shaped structure. If, however, the pump is out of control due to a control or electrical failure, a higher vacuum level than intended is reached, the valve opens and releases the vacuum. The vacuum is then not only released to a level marginally lower than the pressure release threshold but to the significantly lower pressure hold threshold. This behavior is achieved by means of the mechanical properties of the deformable dome-shaped structure which, once deformed, only recovers to its original shape if the pressure drops below the pressure hold threshold as indicates in Fig. 4. The higher pressure level generated by a conventional valve as indicated in Fig. 5, may not allow the user to pull off the breast pump device from the breast to prevent harm.

Alternatively, the valve according to the present invention also allows being used in order to intentionally generate a fluctuating vacuum level. Such a fluctuating vacuum level could, e.g., be used to operate a breast pump device with a fluctuating vacuum behavior mimicking the sucking cycle of an infant. The fluctuating vacuum level is generally generated by combination of pump and solenoid valve, but the valve according to the present invention may be used as an alternative to the solenoid valve. The pressure hold threshold and pressure release threshold depend on the mechanical properties and dimensions of the valve according to the present invention. An example for reasonable thresholds for this application area may be a pressure hold threshold at about 250 mmHg and a low pressure release threshold in the range of 0-90 mmHg. It may be further advantageous to provide means, e.g. a mechanical plug-in construction, for adjusting the pressure release threshold depending on the preferences of a user or medical requirements.

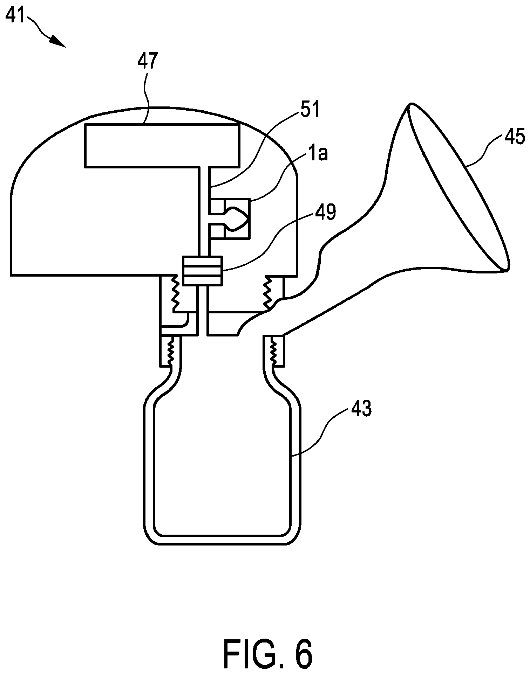

In Fig. 6, there is illustrated a breast pump device 41 for extracting milk from the breast of a lactating woman. The breast pump device 41 comprises a milk reservoir 43 wherein the extracted milk is collected. There is further comprised a funnel 45, i.e. a cushion structure, connected to the milk reservoir 43 which receives the breast of a woman. A vacuum pump 47 generates an underpressure in the milk reservoir 43 and is connected thereto via a diaphragm 49. This diaphragm 49 prevents milk from being sucked out of the milk reservoir 43 into the vacuum pump 47. The pump 47 is connected to the safety valve 1a and the milk reservoir 43 through a tube 51, e.g. a silicone tube. There is further comprised a valve 1a according to an embodiment of the present invention as described above. The pressed pump device 41 may be operated mechanically by hand or foot movements or electronically mains-powered or battery-powered. The breast pump device 41 may comprise additional components such as a one way valve at the inlet of the pump 47 or an additional valve or tank structure for optimizing the generated vacuum level or further components.

Generally, the invention can be applied in different kinds of pump devices for transporting a medium from a first system connected to its inlet to a second system connected to its outlet, wherein at least one valve as described above is interposed between at least one of the first and/or the second system and the inlet and/or outlet of the pump for preventing a backflow of the transported medium through if the pump.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. Valve (1) for controlling the flow of a medium from a first system (24) to a second system (26) comprising
a deformable dome-shaped structure (3) forming a reservoir (25) and including a flow path opening (9);
a valve cover (5) including a first connector opening (15) to connect the valve (1) to the first system (24) and a contact element (23) for interacting with the flow path opening (9) to open and close it; and
a valve base (7) being airtightly connected to the valve cover (5) and to the dome-shaped structure (3) and including a second connector opening (17) to connect the valve (1) to the second system (26) and being coupled to the reservoir (25), wherein the deformable dome-shaped structure (3) is configured such that in an open state of the valve (1) the deformable dome-shaped structure (3) is deformed and the flow path opening (9) is not connected to the contact element (23) to allow a medium to flow from the first system (24) to the second system (26) through the first (15) and second (17) connector openings and the flow path opening (9); and
in a closed state of the valve (1) the deformable dome-shaped structure (3) is in its original dome shape so that the flow path opening (9) is connected to the contact element (23) and thus closed
and **characterized in that**
the deformable dome-shaped structure (3) comprises a hemisphere part (11) with a zenith and wherein the flow path opening (9) is located in the zenith of the hemisphere part (11) of the deformable dome-shaped structure (3).

2. Valve according to claim 1, wherein
the deformable dome-shaped structure (3) is deformed and the flow path opening (9) is not connected to the contact element (23) if the pressure difference between the first system (24) and the second system (26) is above a pressure release threshold (p1); and
the deformable dome-shaped structure (3) is in its original dome shape and the flow path opening (9) is in contact with the contact element (23) if the pressure difference between the first system (24) and the second system (26) is below a pressure hold threshold (p2).

3. Valve according to claim 1, wherein
the deformable dome-shaped structure (3) is deformed and the flow path opening (9) is disconnected from the contact element (23) when the pressure difference between the first system (24) and the second system (26) exceeds a pressure release threshold (p1); and
the deformable dome-shaped structure (3) recovers to its original dome shape and the flow path opening (9) is in contact with the contact element (23) when the pressure difference between the first system (24) and the second system (26) falls below a pressure hold threshold (p2).

4. Valve according to claim 3, wherein the pressure release threshold (p1) is higher than the pressure hold threshold (p2) and the valve (1) is configured to provide a switching hysteresis.

5. Valve according to claim 4, wherein the pressure release threshold (p1) is between 250 mmHg and 450 mmHg, preferably between 330 mmHg and 360 mmHg, and/or the pressure hold threshold (p2) is between 50 mmHg and 200 mmHg, preferably between 90 mmHg and 120 mmHg.

6. Valve according to claim 1, wherein the dome-shaped structure (3) comprises a polymer or elastomer material, preferably silicone or silicone rubber.

7. Valve according to claim 1, wherein the dome-shaped structure (3) includes a cylinder part (13) and a hemisphere part (11), the hemisphere part (11) comprising the flow path opening (9) and having a diameter bigger or equal to the diameter of the cylinder part (13).

8. Valve according to claim 7, wherein the valve base (7) comprises a cylinder-shaped dome connection appendix (19) for receiving the cylinder part (13) of the dome-shaped structure (3) and airtightly sealing the connection.

9. Valve according to claim 1, wherein the contact element (23) includes a flat surface area at least as big as the flow path opening (9) in the deformable dome-shaped structure (3) and arranged to seal the flow path opening (9) when pressed against the flat surface area.

10. Valve according to claim 1, wherein the first connector opening (15) connecting the valve (1) to the first system (24) is represented by four venting holes arranged in a square around the contact element.

11. Valve according to claim 1, wherein the second connector opening (17) connecting the valve (1) to the second system (26) is represented by a cylinder-shaped system connection appendix connecting the reservoir (25) to the second system (26).

12. Valve according to claim 1, wherein valve cover (5) and valve base (7) are integrally formed.

13. Pump device (47) for transporting a medium from a first system (24) connected to its inlet to a second system (26) connected to its outlet, wherein at least one valve (1) according to claim 1 is interposed between at least one of the first system (24) and the inlet of the pump and the second system (26) and the outlet of the pump (47).

14. Breast pump device (41) for extracting milk from the breast of a lactating woman comprising
a milk reservoir (43) for collecting the extracted milk;
a funnel (45) connected to the milk reservoir for receiving a breast of a women;
a vacuum pump (47) for generating an underpressure in the milk reservoir (43); and
a valve (1) according to claim 1 connected to the milk reservoir (43) for preventing the pressure applied to the breast of the woman from exceeding a pressure release threshold (p1) and for providing a pressure fluctuation between the pressure release threshold (p1) and the pressure hold threshold (p2) mimicking the sucking cycle of a infant.

## Patentansprüche

1. Ventil (1) zur Steuerung der Strömung eines Mediums von einem ersten System (24) zu einem zweiten System (26), umfassend
eine verformbare kuppelförmige Struktur (3), die einen Behälter (25) bildet und eine Strömungspfadöffnung (9) enthält;
eine Ventilabdeckung (5), die eine erste Anschlussöffnung (15) zum Anschließen des Ventils (1) an das erste System (24) und ein Kontaktelement (23) zum Zusammenwirken mit der Strömungspfadöffnung (9) enthält, um sie zu öffnen und zu schließen; und
einen Ventilsockel (7), der luftdicht mit der Ventilabdeckung (5) und mit der kuppelförmigen Struktur (3) verbunden ist, und eine zweite Anschlussöffnung (17) zum Anschließen des Ventils (1) an das zweite System (26) enthält, und mit dem Behälter (25) verbunden ist, wobei die verformbare kuppelförmige Struktur (3) konfiguriert ist, sodass
die verformbare kuppelförmige Struktur (3) in einem offenen Zustand des Ventils (1) verformt ist, und die Strömungspfadöffnung (9) nicht mit dem Kontaktelement (23) verbunden ist, um es einem Medium zu erlauben, vom ersten System (24) durch die erste (15) und zweite (17) Anschlussöffnung und die Strömungspfadöffnung (9) zum zweiten System (26) zu strömen; und
die verformbare kuppelförmige Struktur (3) in einem geschlossenen Zustand des Ventils (1) in ihrer ursprünglichen Kuppelform ist, sodass die Strömungspfadöffnung (9) mit dem Kontaktelement (23) verbunden und somit geschlossen ist,
und **dadurch gekennzeichnet, dass** die verformbare kuppelförmige Struktur (3) einen halbkugelförmigen Teil (11) mit einem Scheitelpunkt umfasst, und wobei sich die Strömungspfadöffnung (9) am Scheitelpunkt des halbkugelförmigen Teils (11) der verformbaren kuppelförmigen Struktur (3) befindet.

2. Ventil nach Anspruch 1, wobei
die verformbare kuppelförmige Struktur (3) verformt ist und die Strömungspfadöffnung (9) nicht mit dem Kontaktelement (23) verbunden ist, wenn der Druckunterschied zwischen dem ersten System (24) und dem zweiten System (26) über einem Druckfreigabegrenzwert (p1) liegt;
und
die verformbare kuppelförmige Struktur (3) in ihrer ursprünglichen Kuppelform ist und die Strömungspfadöffnung (9) in Kontakt mit dem Kontaktelement (23) ist, wenn der Druckunterschied zwischen dem ersten System (24) und dem zweiten System (26) unter einem Druckhaltegrenzwert (p2) liegt.

3. Ventil nach Anspruch 1, wobei
die verformbare kuppelförmige Struktur (3) verformt ist und die Strömungspfadöffnung (9) von dem Kontaktelement (23) getrennt ist, wenn der Druckunterschied zwischen dem ersten System (24) und dem zweiten System (26) einen Druckfreigabegrenzwert (p1) überschreitet; und
die verformbare kuppelförmige Struktur (3) wieder ihre ursprüngliche Kuppelform zurückgewinnt und die Strömungspfadöffnung (9) in Kontakt mit dem Kontaktelement (23) ist, wenn der Druckunterschied zwischen dem ersten System (24) und dem zweiten System (26) unter einen Druckhaltegrenzwert (p2) abfällt.

4. Ventil nach Anspruch 3, wobei der Druckfreigabegrenzwert (p1) größer ist, als der Druckhaltegrenzwert (p2) und das Ventil (1) konfiguriert ist, um eine Umschalthysterese bereitzustellen.

5. Ventil nach Anspruch 4, wobei der Druckfreigabegrenzwert (p1) zwischen 250 mmHg und 450 mmHg, vorzugsweise zwischen 330 mmHg und 360 mmHg liegt, und/ oder der Druckhaltegrenzwert (p2) zwischen 50 mmHg und 200 mmHg, vorzugsweise zwischen 90 mmHg und 120 mmHg liegt.

6. Ventil nach Anspruch 1, wobei die verformbare kuppelförmige Struktur (3) ein Polymer- oder Elastomer-Material, vorzugsweise Silikon oder Silikongummi umfasst.

7. Ventil nach Anspruch 1, wobei die verformbare kuppelförmige Struktur (3) ein zylindrisches Teil (13) und ein halbkugelförmiges Teil (11) enthält, wobei das halbkugelförmige Teil (11) die Strömungspfadöffnung (9) umfasst und einen Durchmesser größer oder gleich dem Durchmesser des zylindrischen Teils (13) aufweist.

8. Ventil nach Anspruch 7, wobei der Ventilsockel (7) einen zylinderförmigen Kuppelverbindungsanhang (19) zur Aufnahme des zylindrischen Teils (13) der kuppelförmigen Struktur (3) und zum luftdichten Verschließen der Verbindung umfasst.

9. Ventil nach Anspruch 1, wobei das Kontaktelement (23) einen flachen Oberflächenbereich mindestens so groß wie die Strömungspfadöffnung (9) in der verformbaren kuppelförmigen Struktur (3) enthält und angeordnet, um die Strömungspfadöffnung (9) zu verschließen, wenn sie gegen den flachen Oberflächenbereich gedrückt wird.

10. Ventil nach Anspruch 1, wobei die erste Anschlussöffnung (15), welche das Ventil (1) an das erste System (24) anschließt, durch vier Belüftungslöcher repräsentiert wird, die in einem Quadrat um das Kontaktelement herum angeordnet sind.

11. Ventil nach Anspruch 1, wobei die zweite Anschlussöffnung (17), welche das Ventil (1) an das zweite System (26) anschließt, durch einen zylinderförmigen Systemverbindungsanhang repräsentiert wird, der den Behälter (25) an das zweite System (26) anschließt.

12. Ventil nach Anspruch 1, wobei die Ventilabdeckung (5) und der Ventilsockel (7) in einem Stück gebildet werden.

13. Pumpvorrichtung (47) zum Transportieren eines Mediums von einem ersten System (24), das an ihren Einlass angeschlossen ist, zu einem zweiten System (26), das an ihren Auslass angeschlossen ist, wobei zumindest ein Ventil (1) nach Anspruch 1 zwischen mindestens einem aus dem ersten System (24) und dem Einlass der Pumpe und dem zweiten System (26) und dem Auslass der Pumpe (47) zwischengeschaltet ist.

14. Brustpumpenvorrichtung (41) zum Extrahieren von Milch aus der Brust einer stillenden Frau, umfassend
einen Milchbehälter (43) zum Sammeln der extrahierten Milch;
einen Trichter (45), der an den Milchbehälter angeschlossen ist, zum Aufnehmen einer Brust einer Frau;
eine Vakuumpumpe (47) zum Erzeugen eines Unterdrucks in dem Milchbehälter (43); und
ein Ventil (1) nach Anspruch 1, das an den Milchbehälter (43) angeschlossen ist, um zu verhindern, dass der Druck, der an der Brust der Frau angelegt wird, einen Druckfreigabegrenzwert (p1) überschreitet, und um eine Druckschwankung zwischen dem Druckfreigabegrenzwert (p1) und dem Druckhaltegrenzwert (p2) zur Nachahmung des Saugzyklus eines Säuglings bereitzustellen.

## Revendications

1. Soupape (1) pour réguler l'écoulement d'un milieu d'un premier système (24) vers un second système (26) comprenant
une structure en forme de dôme déformable (3) formant un réservoir (25) et comprenant une ouverture de trajet d'écoulement (9) ;
un couvercle de soupape (5) comprenant une première ouverture de raccordement (15) pour raccorder la soupape (1) au premier système (24) et un élément de contact (23) pour interagir avec l'ouverture de trajet d'écoulement (9) pour ouvrir et fermer celle-ci ; et
une base de soupape (7) raccordée de façon étanche à l'air au couvercle de soupape (5) et à la structure en forme de dôme (3) et comprenant une seconde ouverture de raccordement (17) pour raccorder la soupape (1) au second système (26) et accouplée au réservoir (25), dans lequel la structure en forme de dôme déformable (3) est configurée de sorte que
dans un état ouvert de la soupape (1) la structure en forme de dôme déformable (3) est déformée et l'ouverture de trajet d'écoulement (9) n'est pas raccordée à l'élément de contact (23) pour permettre à un milieu de s'écouler du premier système (24) vers le second système (26) à travers les première (15) et seconde (17) ouvertures de raccordement et l'ouverture de trajet d'écoulement (9) ; et
dans un état fermé de la soupape (1) la structure en forme de dôme déformable (3) est dans sa forme de dôme d'origine de sorte que l'ouverture de trajet d'écoulement (9) soit raccordée à l'élément de contact (23) et ainsi fermée
et **caractérisée en ce que** la structure en forme de dôme déformable (3) comprend une partie hémisphère (11) dotée d'un zénith et dans laquelle l'ouverture de trajet d'écoulement (9) est située dans le zénith de la partie hémisphère (11) de la structure en forme de dôme déformable (3).

2. Soupape selon la revendication 1, dans laquelle
la structure en forme de dôme déformable (3) est déformée et l'ouverture de trajet d'écoulement (9) n'est pas raccordée à l'élément de contact (23) si la différence de pression entre le premier système (24) et le second système (26) est supérieure à un seuil de libération de pression (p1); et
la structure en forme de dôme déformable (3) est dans sa forme de dôme d'origine et l'ouverture de trajet d'écoulement (9) est en contact avec l'élément de contact (23) si la différence de pression entre le premier système (24) et le second système (26) est inférieure à un seuil de maintien de pression (p2).

3. Soupape selon la revendication 1, dans laquelle
la structure en forme de dôme déformable (3) est déformée et l'ouverture de trajet d'écoulement (9) est déconnectée de l'élément de contact (23) lorsque la différence de pression entre le premier système (24) et le second système (26) dépasse un seuil de libération de pression (p1); et
la structure en forme de dôme déformable (3) récupère sa forme de dôme d'origine et l'ouverture de trajet d'écoulement (9) est en contact avec l'élément de contact (23) lorsque la différence de pression entre le premier système (24) et le second système (26) tombe sous un seuil de maintien de pression (p2).

4. Soupape selon la revendication 3, dans laquelle le seuil de libération de pression (p1) est supérieur au seuil de maintien de pression (p2) et la soupape (1) est configurée pour fournir une hystérésis de commutation.

5. Soupape selon la revendication 4, dans laquelle le seuil de libération de pression (p1) est compris entre 250 mmHg et 450 mmHg, de préférence entre 330 mmHg et 360 mmHg, et/ou le seuil de maintien de pression (p2) est compris entre 50 mmHg et 200 mmHg, de préférence entre 90 mmHg et 120 mmHg.

6. Soupape selon la revendication 1, dans laquelle la structure en forme de dôme (3) comprend un matériau polymère ou élastomère, de préférence de la silicone ou du caoutchouc de silicone.

7. Soupape selon la revendication 1, dans laquelle la structure en forme de dôme (3) comprend une partie cylindre (13) et une partie hémisphère (11), la partie hémisphère (11) comprenant l'ouverture de trajet d'écoulement (9) et présentant un diamètre supérieur ou égal au diamètre de la partie cylindre (13).

8. Soupape selon la revendication 7, dans laquelle la base de soupape (7) comprend un appendice de raccordement de dôme en forme de cylindre (19) pour recevoir la partie cylindre (13) de la structure en forme de dôme (3) et assurer l'étanchéité à l'air du raccordement.

9. Soupape selon la revendication 1, dans laquelle l'élément de contact (23) comprend une surface plane au moins aussi grande que l'ouverture de trajet d'écoulement (9) dans la structure en forme de dôme déformable (3) et agencée pour assurer l'étanchéité de l'ouverture de trajet d'écoulement (9) lorsqu'elle est pressée contre la surface plane.

10. Soupape selon la revendication 1, dans laquelle la première ouverture de raccordement (15) raccordant la soupape (1) au premier système (24) est représentée par quatre trous d'aération agencés sous une forme carrée autour de l'élément de contact.

11. Soupape selon la revendication 1, dans laquelle la seconde ouverture de raccordement (17) raccordant la soupape (1) au second système (26) est représentée par un appendice de raccordement de système en forme de cylindre raccordant le réservoir (25) au second système (26).

12. Soupape selon la revendication 1, dans laquelle un couvercle de soupape (5) et une base de soupape (7) sont formés d'un seul tenant.

13. Dispositif de pompe (47) pour transporter un milieu d'un premier système (24) raccordé à son admission à un second système (26) raccordé à son évacuation, dans lequel au moins une soupape (1) selon la revendication 1 est disposée entre le premier système (24) et l'admission de la pompe ou le second système (26) et l'évacuation de la pompe (47).

14. Dispositif de tire-lait (41) pour extraire du lait de la poitrine d'une femme allaitante comprenant
un réservoir de lait (43) pour collecter le lait extrait ;
un entonnoir (45) raccordé au réservoir de lait pour recevoir le sein d'une femme ;
une pompe à vide (47) pour générer une pression négative dans le réservoir de lait (43) ; et
une soupape (1) selon la revendication 1 raccordée au réservoir de lait (43) pour empêcher la pression appliquée au sein de la femme de dépasser un seuil de libération de pression (p1) et pour fournir une fluctuation de pression entre le seuil de libération de pression (p1) et le seuil de maintien de pression (p2) imitant le cycle de succion d'un nourrisson.
